# EUROPEAN PATENT APPLICATION

(11) **EP 4 494 480 A1**
(43) Date of publication of application: **22.01.2025**
(21) Application number: 24184904.1
(22) Date of filing: 27.06.2024
(51) Int. Cl.: A23L 2/39, A23L 29/20, A23L 33/17, A23L 33/19, A23L 33/00, A61P 3/04, A23J 3/08, A23L 29/238, A23L 33/125, A23L 33/15, A23L 33/185, A23L 33/21

(54) **HIGH-PROTEIN COMPOSITION IN POWDER FORM**

(30) Priority: 17.07.2023 IT 202300014946
(71) Applicant: Laboratorio della Farmacia S.p.A., 30020 Quarto d'Altino VE (IT)
(72) Inventor: Farinon, Massimo, 30020 Quarto d'Altino VE (IT); Citton, Filippo, 30020 Quarto d'Altino VE (IT)
(74) Representative: Perani & Partners S.p.A.

(57) **Abstract**

High-protein composition in powder form, comprising
- proteins in a concentration range from about 15% to 85% by weight, said proteins including or consisting of milk protein isolate (MPI),
- fibers in a concentration range from about 2% to 14% by weight, said fibers including or consisting of resistant dextrins and/or polydextrose,
- thickeners in a concentration range from about 0.5% to 8% by weight, the thickeners including or consisting of Tara gum,
in combination with any excipients and/or diluents.

## Description

### FIELD OF THE INVENTION

The present invention falls into the technical field of food compositions in powder form, characterized by a high protein and fiber content and conceived for subjects treated with low-calorie diet.

### BACKGROUND ART

Hyper-protein diet denotes a particular dietary regimen characterized by a higher consumption of foods containing proteins and fats compared to those made up of carbohydrates, both simple and complex, the use of which is very limited.

According to the guidelines for proper nutrition developed by the Italian Society of Human Nutrition ("Società Italiana di Nutrizione Umana", SINU), in a healthy and balanced diet the percentage of protein should be around 12-15% of the kilocalories introduced with food every day. The remaining content should be mostly made up of carbohydrates (45-60%) and secondarily from fats (25-35%). In hyper-protein diets, this distribution is modified and the protein being consumed reaches higher percentages.

Proteins are macromolecules comprising one or more long chains consisting of amino acids, the sequence thereof resulting in proteins folding in a specific three-dimensional structure, which is responsible for the biological activity of the same.

Once formed, proteins exist only for a certain period in the body, and are then degraded and recycled by the cellular mechanism through the process of protein turnover. The lifespan of a protein is measured in terms of half-life: these macromolecules can exist for minutes or years, with an average lifespan of 1-2 days in mammalian cells.

Under normal (physiological) conditions, the main task of proteins is to provide amino acids for building and renewing tissues. In humans, proteins are needed in the diet to provide so-called "essential" amino acids, i.e. those that cannot be synthesized by the body and which include: L-Valine, L-Leucine, L-Isoleucine, L-Tryptophan, L-Methionine, L-Phenylalanine, L-Threonine, L-Lysine.

Amino acids participate only to a negligible extent in the production of energy. This function becomes, instead, prevalent during prolonged fasting, long-term demanding physical activity, and in all situations where the main sources of energy, i.e. carbohydrates, are reduced or even eliminated, as occurs exactly in high-protein diets.

A hyper-protein diet is characterized by a very high protein consumption, exceeding by far the percentages recommended by the guidelines, and is associated with a strong reduction in carbohydrate consumption. The underlying idea is that by significantly reducing the amount of carbohydrate and greatly increasing the amount of protein, the body is induced to use storage fat to produce energy. This, however, only occurs when the hyper-protein diet is also a low-calorie diet, since the amount of carbohydrate and fat introduced is not sufficient to meet the body's energy needs, and therefore the body uses protein and storage fat to produce energy. Otherwise, the excess amount of protein is used through a metabolic pathway (gluconeogenesis) which leads to the formation of glucose, and this can promote the formation of new storage fat.

In order to facilitate the *compliance* of subjects on a hyper-protein diet or to provide nutritional snacks, also suitable for those who are engaged in sport activities, food and pharmaceutical companies have developed products with a high hyper-protein content, which could meet these specific market needs.

Based on current legislation, Regulation (EC) No. 1924/2006, products in which at least 20% of the energy value of the food is provided by proteins are defined as rich in proteins or high-protein products.

Milk protein is very similar to the amino acid composition the World Health Organization (WHO) considers as ideal.

**Table 1. Amino acid composition of the Ideal Protein according to WHO.**

| | | | | | |
|---|---|---|---|---|---|
| L-Leucine | 206.9 | mg | L-Phenylalanine | 197.49 | mg |
| L-Valine | 109.72 | mg | L-Threonine | 103.45 | mg |
| L-Isoleucine | 87.77 | mg | L-Methionine | 78.37 | mg |
| L-Lysine | 181.82 | mg | L-Tryptophan | 34.48 | mg |

Milk protein consists of whey protein and casein.

Milk whey protein accounts for 20% of the total protein in milk. Milk whey, the watery portion of milk, is typically obtained as a by-product of dairy processing, and contains all 8 essential amino acids. Research attributes many benefits to the milk whey protein. It can aid in weight loss efforts and can aid in reducing cholesterol. When used as a food supplement, milk whey protein is rapidly absorbed and metabolized by the body. Researches proved that milk whey protein supplementation can aid in the development of lean muscle mass in athletes. Many athletes and workout lovers report positive results from consuming milk whey protein-based products before, during or immediately after a workout.

As such, milk whey protein doesn't have a pleasant taste; therefore, when used as an ingredient in protein drinks, nutrition bars, etc., flavors and sweeteners are typically included to compensate for the unpleasant taste.

Casein accounts for 80% of the total protein in milk. It is a complete protein as well, which contains all the essential amino acids. As an ingredient in food supplements, this protein is typically found in a concentrated form known as micellar casein. Casein aids in muscle recovery and acts to prevent the breakdown of muscle tissue; it is metabolized much more slowly by the body than milk whey; it stimulates the long-term muscle repair and growth. The cellular structure of casein causes it to remain in the stomach for a prolonged period, thereby providing a sense of satiety. Using casein in the morning or before going to bed or whenever there is no food at hand can aid in keeping hunger at bay.

WO2013/148685 describes a food preparation containing at least pea protein possibly in combination with other protein sources; the use of pea protein has the advantage of giving the composition a certain *texture,* besides having beneficial effects on muscle growth, blood sugar control or blood glucose modulation. In one embodiment, pea protein is associated with milk protein concentrate, where plant protein constitutes between 20% and 50% of the total protein, and milk protein concentrate constitutes between 50% and 80% by weight of the total protein. The preparation further contains carbohydrates, in concentrations varying between 10% and 25% by weight, and selectable from maltodextrins, corn maltodextrins, Fibersol^{®}. The nutritional preparation further includes a fiber capable of modulating the viscosity of the product; among the ingredients suitable for this purpose are guar gum, locust bean gum, tragacanth gum, karaya gum, acacia gum, xanthan gum, galactomannans.

CN110326780A describes a food supplement that serves as a meal replacement and has a hypoglycemic effect for diabetic patients. It contains 20-55% protein; 8-20% fat; 0.1-5% plant extracts. The dietary fiber generally constitutes 8-20% of the food supplement being discussed, with resistant dextrins being mentioned among the dietary fibers suitable for this purpose. Protein can be of plant origin (soybean, pea, rice) or dairy origin (milk protein concentrate, whey protein isolate). Milk protein concentrate is among the preferred ones since it contains about 80% of slow-digesting casein, which stabilizes blood glucose levels, gives a sense of satiety, and prevents large blood glucose fluctuations after meals. The product can contain chewable particles such as, for example, freeze-dried fruit pieces.

CN111011862 describes an edible composition in powder form, useful for the preparation of low glycemic index foods. The composition comprises isomaltulose (10-16%), resistant dextrin (4-9%). The edible composition can be used, in a proportion of 14-63%, for the preparation of a nutritional product powder further comprising a thickening agent in a concentration between 0.2-12%. Thickening agents suitable for this purpose are carrageenans, xanthan gum, gellan, locust bean gum, microcrystalline cellulose, sodium alginate, and soluble soy polysaccharides. The nutritional product powder can comprise proteins, such as whey protein concentrates, whey protein isolates, soy protein isolates, soy protein concentrates, milk protein concentrates.

WO2015/148384 describes a food supplement comprising proteins, polyunsaturated fatty acids, vitamins, minerals and fibers, wherein the proteins include at least milk whey protein concentrate and milk protein concentrate, wherein the ratio of whey protein concentrate to milk protein concentrate varies between 70:30 and 90:10. The protein content (as the sum of whey protein and milk protein) varies between 55-65%. Modified corn dextrins are encompassed as a suitable source of fiber. The food supplement being discussed can contain fiber in a content varying between about 8% and 30% by weight. The preparation is conceived to increase the nutritional value of foods or drinks.

WO2007/108827 describes a food supplement comprising, as a source of protein, milk protein isolate and/or rapeseed plant proteins. The supplement further comprises fat and carbohydrate, among which tapioca dextrins are envisaged. The supplement is conceived for those having specific nutritional needs, such as the need of muscle mass recovery.

WO2021/188791 The document describes a formulation for daily nutrition, containing a mix of proteins, fibers, and optionally fats. The formulation can be used, for example, to control blood glucose, control digestive health, control body weight, improve the sense of satiety, increase insulin resistance, promote a healthy gut microbiome, and support digestive health. The formulations can be made in powder, capsule or tablet form (possibly to be reconstituted in water before consumption), but also in snack or drink form. The formulations contain dietary fibers including starches, beta-glucans, and resistant dextrins. The formulation can also contain a vitamin mix and a thickening agent which, by way of example, can consist of guar gum or xanthan gum.

### Prior art problem

Current commercially available high-protein powder products show the following disadvantages:
- in most cases, they consist of milk whey protein and, therefore, of rapidly absorbed protein;
- they have poor or no fiber content;
- they have a viscosity similar to water when reconstituted (in water). Such a characteristic is undesirable in hyper-protein products based on milk protein, which the consumer expects to have instead a fluid/semi-solid appearance. Furthermore, products of this type, when heated in the microwave, tend to form a mass of caseous consistency which is undesirable to the consumer's eye and palate;
- they contain exclusively flavors, and rarely freeze-dried fruits or vegetables in the form of powder or pieces;
- when ingested, they do not noticeably reduce the sense of hunger.

Therefore, there exists a need for high-protein products in powder form that, while leading to a reduced calorie intake, satisfy the sense of hunger by virtue of the rheology of the product (both hot and cold) and induce a sense of satiety by virtue of their nutritional content, reducing the absorption of lipids and cholesterol, and slowing the absorption of sugars.

### SUMMARY OF THE INVENTION

The Applicant has now formulated a high-protein composition, capable of meeting the needs of the market, by solving the prior art problems discussed above.

An object of the present invention is a high-protein composition in powder form, comprising
- proteins in a concentration range from 15% to 85% by weight, said proteins comprising or consisting of milk protein isolate (MPI),
- fibers in a concentration range from 2% to 14% by weight, said fibers comprising or consisting of resistant dextrins and/or polydextrose,
- thickeners in a concentration range from 0.5% to 8% by weight, the thickeners comprising or consisting of Tara gum,
in combination with any excipients and/or diluents.

The powder composition of the invention is preferably usable as is, in the form of a food supplement, snack or meal replacement, to be dispersed in water or milk before consumption; alternatively, it can be processed together with further food ingredients in order to obtain finished food products (such as, for example, bars, yogurt, drinks).

Therefore, a further object of the application relates to the uses of the hyper-protein composition in making food supplements or foods; in the treatment of subjects on low-calorie diet therapy (i.e., overweight subj ects); in the nutrition or food supplementation of healthy subjects on a balanced diet.

### Advantages of the invention

The composition according to the invention is characterized by a high content of protein and fiber that negligibly affect blood sugar and provide a successful satiating effect.

Despite the high protein content, the sizing of the composition in single-dose sachets or single food servings is particularly advantageous: the weight of the finished product - which, in the preferred embodiment, varies between about 15 g and 33 g - is not excessively high, making therefore packaging easier and more economically sustainable.

Thanks to its components and high viscosity it is able to reduce the sense of hunger in patients treated with low-calorie diet.

The high protein content also reduces the loss of lean mass and allows the individual, under certain dietary conditions, to enter ketosis.

Resistant dextrins as the main or exclusive ingredient of the fiber fraction proved to be particularly advantageous for the purposes of the invention since they are easily soluble in an aqueous medium; they have a neutral taste which does not affect the flavor of the finished product; they do not impact on the individual's blood sugar and have a poor FODMAP (Fermentable Oligosaccharides, Disaccharides, Monosaccharides and Polyols) effect, i.e. they are not easily fermentable in the small intestine, thus avoiding characteristic disorders of fiber intake such as bloating, meteorism, spasticity, constipation, and diarrhea.

Finally, Tara gum, as the main or exclusive ingredient of the thickener fraction of the composition, proved to be ideal for obtaining the desired viscosity and appearance characteristics, both under hot and cold conditions.

### DETAILED DESCRIPTION OF THE INVENTION

A detailed description of the invention and the preferred embodiments thereof is provided below in the document. Note that the fact that the description has been organized into sub-chapters does not mean that the information contained in each sub-chapter is isolated or cannot be combined with that contained in the other sub-chapters or, more generally, with other information contained in the text of the patent application.

### High-protein composition

As already remarked above, the object of the invention is a hyper-protein composition in powder form, comprising
- proteins in a concentration range from about 15% to 85%, preferably from about 15% to 82% by weight, said proteins comprising or consisting of milk protein isolate (MPI),
- fibers in a concentration range from about 2% to 14%, preferably from about 2% to 13% by weight, said fibers comprising or consisting of resistant dextrins or polydextrose,
- thickeners in a concentration range from about 0.5% to 8%, preferably from about 0.5% to 5% by weight, the thickeners comprising or consisting of tara gum,
in combination with any excipients and/or diluents.

According to a preferred embodiment, the high-protein composition comprises
- proteins in a concentration range from about 30% to 81% by weight, said proteins comprising or consisting of milk protein isolate (MPI),
- fibers in a concentration range from about 3% to 10% by weight, said fibers comprising or consisting of resistant dextrins or polydextrose,
- thickeners in a concentration range from about 1% to 6% by weight, the thickeners comprising or consisting of tara gum,
in combination with any excipients and/or diluents.

Still preferably, the high-protein composition comprises
- proteins in a concentration range from about 60% to 81% by weight, said proteins comprising or consisting of milk protein isolate (MPI),
- fibers in a concentration range from about 5% to 10% by weight, said fibers comprising or consisting of resistant dextrins or polydextrose,
- thickeners in a concentration range from about 2% to 5% by weight, the thickeners comprising or consisting of tara gum,
in combination with any excipients and/or diluents.

Note that, preferably, the high-protein composition has to be taken upon reconstitution in water, milk or other aqueous drink. Preferably, the reconstituted product obtained by mixing the high-protein composition with water is characterized by a viscosity between 2.600 mPa*s and 4.700 mPa*s, measured with an AMETEK BROOKFIELD Viscometer, following the method reported in Example 7.

Note that, advantageously, this viscosity is maintained or increased in case the reconstituted product is heated to temperatures lower than 100°C, preferably lower than 80°C, preferably between 60°C and 70°C, approximately. The increase in viscosity is attributable to the fact that the heat causes the Tara gum to rehydrate more quickly. In case an increase in viscosity is noticed under heating, preferably the viscosity of the reconstituted product obtained by mixing the high protein content composition with water varies between 13.000 mPa*s and 17.000 mPa*s.

Preferably, the high-protein composition is characterized by low fat and carbohydrate content, being preferably intended for use in food supplementation or nutrition (as food) of subjects treated with low-calorie diet.

Preferably, the high-protein composition contains a carbohydrate titer of less than 10% by weight, based on the total weight of the composition, preferably between 2% and 9% by weight.

Preferably, the high-protein composition is characterized by a fat content of less than 7% by weight, based on the total weight of the composition, preferably between 1% and 5% by weight.

### Protein

As already stated, the high-protein composition comprises a protein fraction including or consisting of milk protein isolate (MPI).

Note that, preferably, MPI constitutes between 60% and 100% of the total protein of the high-protein composition, preferably between 70% and 100% of the total protein of the high-protein composition, preferably between 80% and 100% of the total protein of the high-protein composition, preferably between 90% and 100% of the total protein, preferably between 95% and 100% of the total protein of the high-protein composition.

According to a preferred embodiment, the protein fraction of the high-protein composition comprises, in addition to the milk protein isolate, also
- plant-based proteins, preferably selected from soy, bean, pea, lentil and chickpea, pumpkin, hemp, and potato proteins
   and/or
- further animal-based proteins, preferably selected from milk protein concentrate (MPC), egg proteins, white egg proteins.

The products based on milk protein concentrate (including MPC and MPI) are generally obtained by concentrating bovine skim milk so that the finished dry product contains at least 40% protein by weight. The concentration processes used to obtain these products are filtration, dialysis or any other safe and appropriate process that allows all or part of the lactose and minerals to be removed. Note that products based on milk protein concentrate cannot be obtained by combining casein (caseinate) and milk whey protein produced separately.

MPC and MPI are preferably produced by ultrafiltration or diafiltration methods which capture essentially all casein and whey protein contained in the raw material. Preferably, both MPC and MPI are characterized by a casein/milk whey protein ratio equivalent to that of the original milk, and preferably between about 82:18 and 95:5, preferably of about 80:20.

Note that products based on milk protein concentrate are sometimes obtained using microfiltration; this process, however, alters the casein/whey protein ratio compared to that found in milk (and, therefore, does not allow MPC or MPI to be obtained). When microfiltration is used, the resulting product is called Microfiltered Milk Protein (MMP) or Micellar Casein (MC).

Note that, generally, MPC and MPI differ from each other in their nutritional profile. MPC is preferably characterized by a protein content varying between about 39.5% and 85% by weight; a fat content varying between about 1.25% and 2.50% by weight; a lactose content varying between about 5.0% and 52.0% by weight. Instead, MPI is preferably characterized by a protein content between about 85% and 90% by weight; a fat content varying between about 1.25% and 2.50% by weight; and a lactose content varying between about 0.2% and 5%, preferably 0.2% and 1%.

According to the protein content, MPC is classified into MPC 40, MPC 42, MPC 56, MPC 70, MPC 80 and MPC 85. For the purposes of the invention, MPC 80 and 85 are preferably used.

Preferably, for the purposes of making the high-protein composition of the invention, the milk whey protein isolate (MWPI) cannot replace MPI. MWPI actually has an amino acid composition very similar to the WHO ideal protein in Table 1. Based on systematics performed during the product development phase, however, hyper-protein compositions comprising MWPI and a thickener in a concentration between 1-3% (Xanthan Gum), show a liquid appearance of the reconstituted product obtained by dispersion in water; furthermore, when heated in a microwave, the reconstituted product returns a mass with a caseous consistency that is far from the fluid/semi-solid appearance sought for a high-protein preparation based on milk protein. Furthermore, MWPI does not impart the product a well-rounded and consistent aroma, and causes the rapid onset of the sense of hunger, as they are rapidly assimilated proteins. Note, in fact, that caseins and whey protein generate a different effect on the increase of satiety related to time. Milk whey protein acts in the short term: consuming 45 g to 50 g of milk whey protein reduces food intake during a meal consumed within 30-90 minutes after ingesting the protein (Anderson, G. H., & Moore, S. E. (2004). Dietary proteins in the regulation of food intake and body weight in humans. The Journal of nutrition, 134(4), 974S-979S. https://doi.org/10.1093/jn/134.4.974S). Casein, instead, is effective for meals consumed about 3 hours after ingestion of that protein (Moore, S. E. (2004). The effects of milk proteins on the regulation of short-term food intake and appetite in young men. https://tspace.library.utoronto.ca/handle/1807/118791).

According to a preferred embodiment, the high-protein composition of the invention does not contain MWPI.

Note that, for the purposes of the invention, MPC does not constitute a valid substitute for MPI either. Unlike compositions comprising MWPI, compositions comprising MPC and a thickener in a concentration between 1-3% (Xanthan Gum), when dissolved in water, impart the resulting product the desired viscosity (2600 mPa*s - 17.000 mPa*s); the taste of these compositions being slightly sweet, well-rounded and consistent. Such viscosity persists almost unvaried even under hot conditions, and does not form any caseous mass, as noticed using MWPI. MPC, similarly to MPI, contains both milk whey protein and casein, and is therefore able to have an immediate nourishing effect and a prolonged satiating effect, which is very useful for those on a low-calorie diet. However, the protein content provided by MPC is lower than that provided by MPI; the use of MPC as an alternative to MPI, therefore, would lead to a % decrease in the mixed protein content.

Thus, in other words, MPC can be contained in the high-protein composition of the invention preferably in a concentration range from 0% to 40%, preferably from 0% to 30%, preferably from 0% to 20%, preferably from 0% to 10% by weight, preferably from 0% to 5% by weight, based on the weight of total protein.

In case plant proteins are included in addition to MPI, such plant proteins can constitute between about 0% and 55%, preferably between about 0% and 52%, preferably between about 10% and 52%, preferably between 15% and 52 % by weight based on the weight of total protein.

Therefore, MPI is important for making the high-protein composition since, similarly to MPC, it allows to obtain satisfying taste and viscosity, but at the same time it allows to make compositions with a higher protein content, in a single dose/single serving amount which preferably does not exceed 63 g. Furthermore, MPI proves to have greater flowability than MPC.

### Thickening agent

Hydrocolloids or gums are the preferred thickening agents for the purposes of the invention. Still preferably, the thickening agents that can be employed for the purposes of the invention (in addition to Tara gum) are selected from the group consisting of: (i) gums of natural origin, such as gum arabic, locust bean gum, guar gum, xanthan gum, gellan gum; and *(ii)* gums of synthetic origin, such as CMC (CarboxyMethylCellulose), HPMC (HydroxyPropylMethylCellulose), EC (EthylCellulose), other cellulose derivatives known to those skilled in the art; and combinations thereof.

If ingested in large amounts, natural gums can cause abdominal discomfort, as they are fermentable; synthetic gums, such as CMC, can, instead, cause constipation problems if not properly hydrated.

Preferably, Tara gum constitutes between 60% and 100% of the total thickening agents of the high-protein composition, preferably between 70% and 100% of the total thickening agents, preferably between 80% and 100% of the total thickening agents, preferably between 90% and 100% of the total thickening agents, preferably between 95% and 100% of the total thickening agents.

Note that the Applicant has selected Tara gum, among the natural thickeners commercially available, since it allows to impart a natural appearance to the reconstituted product obtained by dispersing the high-protein composition in water, as well as the desired viscosity - both under hot and cold conditions, without impacting on the taste of the finished product.

Xanthan gum does not constitute a valid substitute for Tara gum for the purposes of the invention, since it imparts the reconstituted product obtained by dissolving the high-protein composition in water a synthetic and translucent appearance similar to plastic.

Gum arabic does not constitute a valid substitute for Tara gum for the purposes of the invention, since - when mixed with xanthan gum - it imparts the reconstituted product a translucent appearance that is undesirable in a food product. As such (i.e., not mixed with xanthan gum), gum arabic is not able to impart the reconstituted product the desired viscosity (the reconstituted product remains fluid).

Guar gum does not constitute a valid substitute for Tara gum for the purposes of the invention, since, despite imparting the reconstituted product the desired viscosity, it gives an undesirable translucent effect to a food product and impacts on the taste thereof with an underlying bitter-like characteristic.

### Fiber

The high-protein composition of the invention contains further a fiber fraction. Fiber is essential for a proper nutrition, besides being capable of increasing the satiating effect and balancing bowel function without affecting the rise in blood sugar.

For the purposes of implementing the invention, the high-protein composition preferably comprises soluble fiber and non-fermentable fiber (and thus having a low FODMAP effect), in addition to resistant dextrins and/or polydextrose.

Soluble fiber, which dissolves in water, can aid in reducing blood glucose, triglyceride and cholesterol levels. Foods with soluble fiber include oat flour, chia seeds, walnuts, beans, lentils, apples, and blueberries.

Insoluble fiber, which does not dissolve in water, can aid food moving through the digestive system, promoting regularity and aiding in preventing constipation, and acting as a garbage collector on the intestinal epithelium. Foods with insoluble fiber include whole grain products, especially wheat bran, quinoa, brown rice, legumes, green leafy vegetables such as kale, almonds, walnuts, seeds, and fruits with edible skin, such as pears and apples.

Fermentable fiber serves as food for intestinal bacteria that break it down and ferment it, often producing intestinal problems such as bloating and meteorism.

Non-fermentable fiber is not broken down by bacteria and travels intact to the colon and can increase the bulk, weight, and consistency of stool, making it easier to pass it.

Preferably, fibers suitable for implementing the invention, in addition to resistant dextrins and/or polydextrose, are selected from
- Beta-glucans: Highly fermentable soluble fiber found in oats and barley that is metabolized and fermented in the small intestine. It acts as a prebiotic. It may add bulk to the stool but does not have a laxative effect. It can aid in normalizing the blood glucose and cholesterol levels.
- Inulin, oligosaccharides, pectins, resistant starch, gums.
- combinations thereof.

Polydextrose is a soluble fiber made up of glucose and sorbitol. It can increase stool bulk and have a mild laxative effect. It is advantageous for the purposes of the invention, since it has a sweetening power, while having a minimal effect on glycemia; it aids in improving the consistency, maintaining the moisture or increasing the fiber content.

Resistant dextrins are soluble fibers prepared by controlled partial hydrolysis and repolymerization of the dextrinization process.

Resistant dextrins are important for the purposes of implementing the invention, since they have a neutral taste, are easily soluble in water, do not increase the blood sugar level after intake, and have a low FODMAP effect.

Preferably, the composition of the invention comprises resistant dextrins made from wheat (i.e., obtained from wheat starch) or corn (i.e., obtained from corn starch). Still preferably, the resistant dextrins for the purposes of the invention are those from corn.

According to a preferred embodiment, the resistant dextrins constitute between 50% and 100% by weight based on the total weight of the fiber fraction of the composition of the invention, preferably between 60% and 100%, preferably between 80% and 100%. The remaining part up to 100 of the fiber fraction consists of polydextrose as such, or polydextrose in combination with beta-glucans and/or inulin.

Note that Psyllium, a soluble, non-fermentable fiber extracted from psyllium seeds, does not constitute a valid substitute for resistant dextrins and/or polydextrose, since it is difficult to dissolve in water and imparts an unpleasant, "grassy" taste to the product.

In this regard, the high-protein composition of the invention preferably does not comprise Psyllium.

### Nutraceutical actives

According to a preferred embodiment, the high-protein composition further comprises nutraceutical actives selected from the group consisting of: one or more B-group Vitamins or salts thereof, Vitamin C or salts thereof, E-group Vitamins, A-group Vitamins, one or more D-group Vitamins, amino acids, peptides, plant-based extracts and combinations thereof.

Still preferably, the nutraceutical actives that can be included in the high-protein composition are selected from the group consisting of: Vitamin C, Niacin (Vitamin B3), Vitamin E, Pantothenic Acid (Vitamin B5), Riboflavin (Vitamin B2), Pyridoxine (Vitamin B6), Thiamine (Vitamin B 1), Folic Acid (Vitamin B9), Biotin (Vitamin B8), Vitamin B 12, Vitamin A, Vitamin B3, Chlorophyll and combinations thereof.

Note that, for the purposes of the present invention and for each of the nutraceutical actives listed above, any derivatives thereof suitable for the formulation of a product to be taken orally/enterally, such as the high-protein composition of the invention, are also intended to be included in the scope of protection. By way of non-limiting example, derivatives of nutraceutical actives can be salts, isomers, dimers or precursors thereof.

### Excipients and/or diluents

The high-protein composition of the invention contains excipients and/or diluents commonly used in the pharmaceutical/nutraceutical or food fields.

By way of non-limiting example, the high-protein composition preferably comprises excipients and/or diluents selected from glidant agents (e.g., hydrated silica), preservatives (e.g., citric acid, malic acid), flavors, pH regulators, effervescent agents (such as carbonates or bicarbonates), sweeteners.

Among the possible sweeteners, sucralose and stevia are preferred. Among the possible diluents, erythritol and isomalt are preferred. The person skilled in the field will be able to recognize, based on their knowledge and the peculiarities of the process, which other sweeteners or diluents can be used for the purposes of the invention.

The person skilled in the food and/or pharmaceutical field has no difficulty, based on their knowledge and on the purposes of the invention, in understanding and identifying which types of excipients and/or diluents can be used in the formulation of the high-protein composition.

### Uses of the high-protein composition

According to a preferred embodiment, the high-protein composition can be used to make food supplements or foods fortified with vitamins or mineral salts. Therefore, a further object of the present invention relates to food supplements or foods comprising or consisting of the high-protein composition of the invention.

"Food supplements" are defined by the relevant legislation (Directive 2002/46/EC implemented by the Legislative Decree issued on May 21, 2004, n. 169) as: *"dietary products designed to integrate the normal diet that are a concentrated source of nutrients, such as vitamins and minerals, or of other substances with nutritional or physiological effects, especially but not only amino acids, essential fatty acids, fibres and plant-based extracts, either mono- or multi- compounds, marketed in pre-dosed form".*

"Food" means what is provided in the definition found in art. 2 of EC REG 178/2022, i.e., *"<<Food>> (or* <<*foodstuff*>>) *means any substance or product, whether processed, partially processed or unprocessed, intended to be, or reasonably expected to be ingested by humans.* <<*Food*>> *includes drink, chewing gum and any substance, including water, intentionally incorporated into the food during its manufacture, preparation or treatment* [...]".

Preferably, in case of food supplements manufacturing, the high-protein composition in powder form is used as is, possibly supplementing it with technological ingredients (which can aid in the processing of the powder in nutraceuticals/pharmaceuticals production facilities).

Instead, in case of food preparation, the high-protein composition in powder form can be used as is in order to make a fluid food or an instant drink, after suspending it in water; or it can be mixed with other food ingredients (fresh or preserved) and possibly cooked or heated.

For the purposes of the present invention, food means a preparation that can be used as a meal element or as a meal replacement in the diet of an individual (whether overweight or normal weight). By way of non-limiting example, foods suitable for taking advantage of the inventive high-protein composition are yogurt, desserts, food bars, ice cream, soups, spreadable cheeses, spreadable creams or the likes. The person skilled in the food field will be able, based on their technical knowledge, to identify other foods in which, according to the teachings of the invention, it is possible to include the high-protein composition herein described.

Note that, for the purposes of the present invention, meal replacement means a food product intended to replace a solid food, usually with controlled amounts of calories and nutrients.

Still preferably, the food supplements or foods including or consisting of the high-protein composition can be packaged in single-dose sachets or single food servings.

Preferably, the single doses/single servings comprising or consisting of the high-protein composition of the invention have a weight varying between 10 g and 63 g, preferably between 10 g and 50 g, preferably between 10 g and 40 g, preferably between 10 g and 33 g, preferably between 15 g and 33 g.

According to a preferred embodiment, the single-dose sachets or single food servings are advantageously characterized by a moderate calorie content, preferably between 45 kcal and 120 kcal. Still preferably, food supplements have a calorie content varying between 50 kcal and 90 kcal; foods have a content varying between 90 kcal and 120 kcal.

According to a particularly preferred embodiment, the single dose or single serving of the high-protein composition contains
- MPI in amounts from 10 to 25 g, preferably from 15 to 19 g
- resistant dextrins in amounts from 0.3 to 4 g, preferably from 0.8 to 4 g, preferably from 1 to 3 g
- tara gum in amounts from 0.3 to 4 g, preferably from 0.5 to 4 g.
- nutraceutical actives in amounts from 0.01 g to 0.4 g, wherein the nutraceutical actives are selected from the group consisting of: one or more B-group Vitamins, Vitamin C, Vitamin E, Vitamin A, one or more D-group Vitamins, plant-based extracts and combinations thereof.

According to a preferred embodiment, the single dose or single serving of the high-protein composition contains
- powdered freeze-dried fruits and/or freeze-dried fruit pieces in amounts from 0.4 to 3 g, preferably from 1 to 3 g. Preferably, such freeze-dried fruits are not supported by maltodextrins, starch or glucose.
   and/or
- powdered freeze-dried vegetables and/or freeze-dried vegetable pieces in amounts from 1 to 15 g, preferably from 2 to 15 g, preferably from 2 to 10 g, preferably from 2 to 8 g. Preferably, such freeze-dried vegetables are not supported by maltodextrins or glucose.
   and/or
- chocolate pieces or cocoa powder in amounts from 1 to 10 g, preferably from 1 to 8 g.

According to a preferred embodiment, the high-protein composition is intended for use in the treatment of overweight conditions, as a food supplement or as a food. Still preferably, the high-protein composition is intended for use in the nutrition supplementation or in the nutrition of individuals treated with low-calorie diet (overweight).

According to a different preferred embodiment, the high-protein composition can be used for non-medical purposes, in healthy individuals on a balanced diet, for the purposes of supplementing the nutrition with proteins or as snacks/desserts in one's nutrition.

### EXAMPLES

### 1. High-protein powder to be reconstituted in water or milk. Flavor: Custard

INGREDIENTS: Milk Protein (MPI); Corn soluble fiber (Resistant dextrin); Flavors; Tara gum; Silicon dioxide; Sodium chloride; L-Lysine; L-Tryptophan; Sweetener: Sucralose; L-ascorbic acid (Vit. C); Vanilla powder; DL-Tocopheryl acetate (Vit. E); Nicotinamide (Niacin); Calcium D-pantothenate (Pantothenic Acid); Riboflavin 5-phosphate (Vit. B2); Cholecalciferol (Vit. D); Pyridoxine hydrochloride (Vit. B6); Thiamine hydrochloride (Vit. B 1); Retinyl acetate (Vit. A); Cyanocobalamin (Vit. B 12); Pteroylmonoglutamic acid (Folic Acid); D-Biotin (Biotin)

| **27 g sachet** | | |
|---|---|---|
| Milk protein MPI 90% | 22.00 | g |
| L-Tryptophan | 0.13 | g |
| L-Lysine | 0.27 | g |
| Hydrated silica | 0.41 | g |
| Resistant dextrin | 1.58 | g |
| Tara gum | 1.00 | g |
| Custard flavor | 1.25 | g |
| Sucralose | 0.04 | g |
| Sodium chloride | 0.27 | g |
| Vitamin C | 0.02100 | g |
| Niacin | 0.00333 | g |
| Vitamin E | 0.01088 | g |
| Calcium pantothenate | 0.00138 | g |
| Riboflavin 5 phosphate | 0.000432 | g |
| Pyridoxine hydrochloride | 0.000365 | g |
| Thiamine hydrochloride | 0.000305 | g |
| Folic acid | 0.000048 | g |
| Biotin | 0.0000108 | g |
| Vitamin B12 | 0.000060 | g |
| Vitamin A powd. | 0.000206 | g |
| Vitamin D3 powd. | 0.00043 | g |
| Beta carotene | 0.002 | g |
| Vanilla bean powder | 0.010 | g |
| *TOTAL* | ***27.00*** | ***g*** |

| **AVERAGE NUTRITION FACTS** | | |
|---|---|---|
| Kcal | 87 | |
| Fat | 0.3 | g |
| *saturated* | 0.2 | g |
| Carbohydrate | 0.18 | g |
| *sugars* | 0.18 | g |
| Fiber | 2.46 | g |
| Protein | 19.35 | g |
| Salt | 0.45 | g |

### 2. High-protein powder to be reconstituted in water or milk. Flavor: Cream and strawberry

INGREDIENTS: Milk Protein (MPI); Strawberry powder (4.5%); Cream Powder (4.5%); Flavors; Tara gum; Corn soluble fiber (Resistant dextrin); Silicon dioxide; Sodium chloride; L-Lysine; L-Tryptophan; Acidulants: Citric acid, Malic acid; Sweetener: Sucralose; L-ascorbic acid (Vit. C); DL--Tocopheryl acetate (Vit. E); Nicotinamide (Niacin); Calcium D-pantothenate (Pantothenic Acid); Riboflavin 5-phosphate (Vit. B2); Cholecalciferol (Vit. D); Pyridoxine hydrochloride (Vit. B6); Thiamine hydrochloride (Vit. B1); Retinyl acetate (Vit. A); Cyanocobalamin (Vit. B12); Pteroylmonoglutamic acid (Folic Acid); D-Biotin (Biotin).

| **28.00 g sachet** | | |
|---|---|---|
| Milk protein MPI 90% | 21.26 | g |
| L-Tryptophan | 0.10 | g |
| L-Lysine | 0.2 | g |
| Hydrated silica | 0.44 | g |
| Resistant dextrins | **0.85** | g |
| Tara gum | 1.00 | g |
| Strawberry flavor | 0.52 | g |
| Cream flavor | 0.65 | g |
| Strawberry fruit powder | 1.25 | g |
| Cream 75% powder | 1.25 | g |
| Citric acid | 0.07 | g |
| Malic acid | 0.007 | g |
| Sucralose | 0.07 | g |
| Sodium chloride | 0.3 | g |
| Vitamin C | 0.02100 | g |
| Niacin | 0.00333 | g |
| Vitamin E powd. | 0.01088 | g |
| Calcium pantothenate | 0.00138 | g |
| Riboflavin 5 phosphate | 0.000432 | g |
| Pyridoxine Hydrochloride | 0.000365 | g |
| Thiamine Hydrochloride | 0.000305 | g |
| Folic acid | 0.000048 | g |
| Biotin | 0.0000108 | g |
| Vitamin B12 | 0.000060 | g |
| Vitamin A powd. | 0.000206 | g |
| Vitamin D3 powd. | 0.00043 | g |
| ***TOTAL*** | ***28.00*** | ***g*** |

| **AVERAGE NUTRITION FACTS** | | |
|---|---|---|
| Kcal | 96 | |
| Fat | 1.26 | g |
| *saturated* | 0.82 | g |
| Carbohydrate | 1.01 | g |
| *sugars* | 1.00 | g |
| Fiber | 1.93 | g |
| Protein | 18.83 | g |
| Salt | 0.5 | g |

### 3. High-protein powder to be reconstituted in water. Flavor: Mint and chocolate

INGREDIENTS: Milk protein (MPI); 60% dark chocolate (6.3%): Cocoa Mass, Sugar, Cocoa Butter, Emulsifier (Soy Lecithin), natural Vanilla Extract; Tara gum; Corn soluble fiber (Resistant dextrin); Flavors; Silicon dioxide; Sodium chloride; L-Lysine; L-Tryptophan; Mint leaves (0.2%); Dye: Chlorophyll; Sweetener: Sucralose; L-ascorbic acid (Vit. C); DL--Tocopheryl acetate (Vit. E); Nicotinamide (Niacin); Calcium D-pantothenate (Pantothenic Acid); Riboflavin 5-phosphate (Vit. B2); Cholecalciferol (Vit. D); Pyridoxine hydrochloride (Vit. B6); Thiamine hydrochloride (Vit. B 1); Retinyl acetate (Vit. A); Cyanocobalamin (Vit. B 12); Pteroylmonoglutamic acid (Folic Acid); D-Biotin (Biotin).

| **27 g sachet** | | |
|---|---|---|
| Milk protein MPI 90% | 21.73 | g |
| L-Tryptophan | 0.11 | g |
| L-Lysine | 0.22 | g |
| Hydrated silica | 0.35 | g |
| Resistant dextrins | 0.93 | g |
| Tara gum | 1.00 | g |
| Natural mint flavor | 0.42 | g |
| Peppermint leaves | 0.05 | g |
| Chocolate 60% grains | 1.70 | g |
| Chlorophyll | 0.03 | g |
| Sucralose | 0.03 | g |
| Sodium chloride | 0.24 | g |
| Vitamin C | 0.02100 | g |
| Niacin | 0.00333 | g |
| Vitamin E powder | 0.01088 | g |
| Calcium Pantothenate | 0.00138 | g |
| Riboflavin 5 phosphate | 0.000432 | g |
| Pyridoxine hydrochloride | 0.000365 | g |
| Thiamine hydrochloride | 0.000305 | g |
| Folic acid | 0.000048 | g |
| Biotin | 0.0000108 | g |
| Vitamin B12 | 0.000060 | g |
| Vitamin A powd. | 0.000206 | g |
| Vitamin D3 powd. | 0.00043 | g |
| | ***27.00*** | ***g*** |

| **AVERAGE NUTRITION FACTS** | | |
|---|---|---|
| Kcal | 94 | |
| Fat | 1.01 | g |
| *saturated* | 0.64 | g |
| Carbohydrate | 0.86 | g |
| *sugars* | 0.81 | g |
| Fiber | 1.94 | g |
| Protein | 19.14 | g |
| Salt | 0.43 | g |

### 4. High-protein powder for the preparation of a Banana and Chocolate flavored Dessert

INGREDIENTS: Milk Protein MPI (75.24%); Dark chocolate (5.00%); Banana fruit powder (7.58%); Soluble corn fiber Corn dextrin (3.33%); Tara gum (3.33%); Flavor; Silicon dioxide; Sodium chloride; L-Lysine; L-Tryptophan; Sucralose; L-ascorbic acid (Vit. C); DL-α-Tocopheryl acetate (Vit. E); Nicotinamide (Niacin); Retinyl acetate (Vit. A); Cholecalciferol (Vit. D); Calcium D-pantothenate (Pantothenic Acid); Riboflavin 5-phosphate (Vit. B2); Pyridoxine hydrochloride (Vit. B6); Thiamine hydrochloride (Vit. B1); Cyanocobalamin (Vit. B12); Pteroylmonoglutamic acid (Folic Acid); D-Biotin (Biotin).

| **AVERAGE NUTRITION FACTS:** | | | **1 bag (g)** | | |
|---|---|---|---|---|---|
| | **Per 100 g** | | **30** | **g** | ***NRV (%)*** |
| **Energy** | **1455** | kJ | 437 | kJ | |
| | **348** | kcal | 104 | kcal | |
| **Fat** | **3.16** | g | 0.95 | g | |
| *saturated* | *2.55* | g | *0.77* | g | |
| **Carbohydrate** | **8.80** | g | 2.64 | g | |
| *sugars* | *8.39* | *g* | *2.52* | *g* | |
| *polyols* | | g | *0.00* | *g* | |
| **Fiber** | **7.08** | g | 2.12 | g | |
| **Protein** | **66** | g | 20 | g | |
| **Salt** | **1.69** | g | 0.51 | g | |
| Vitamin C | 46.67 | mg | 14.00 | mg | *17.5* |
| Niacin | 9.33 | mg | 2.80 | mg | *17.5* |
| Vitamin E | 6.80 | mg | 2.04 | mg | *17.0* |
| Pantothenic acid | 3.53 | mg | 1.06 | mg | *17.7* |
| Riboflavin | 0.87 | mg | 0.26 | mg | *18.6* |
| Pyridoxine Hydrochl. | 0.83 | mg | 0.25 | mg | *17.9* |
| Thiamine Hydrochloride | 0.67 | mg | 0.20 | mg | *18.2* |
| Vitamin A | 0.50 | mg | 0.15 | mg | *18.8* |
| Folic acid | 0.13 | mg | 0.040 | mg | *20* |
| Biotin | 0.03 | mg | 0.009 | mg | *18* |
| Vitamin D3 | 0.0030 | mg | 0.0009 | mg | *18* |
| Vitamin B12 | 0.002 | mg | 0.0005 | mg | *20* |

### 5. High-protein powder for the preparation of a Cocoa flavored Dessert

INGREDIENTS: Milk Protein MPI (69.00%); Cocoa powder (17.20%); Tara gum (3.33%); Soluble corn fiber Corn dextrin (1.10%); Flavor; L-Lysine; Silicon dioxide; Sodium chloride; L-Tryptophan; Caramel powder; Sucralose; L-ascorbic acid (Vit. C); DL-α-Tocopheryl acetate (Vit. E); Nicotinamide (Niacin); Retinyl acetate (Vit. A); Cholecalciferol (VIT .D); Calcium D-pantothenate (Pantothenic Acid); Riboflavin 5-phosphate (VIT. B2); Pyridoxine hydrochloride (Vit. B6); Thiamine hydrochloride (Vit. B1); Cyanocobalamin (Vit. B12); Pteroylmonoglutamic acid (Folic Acid); D-Biotin (Biotin).

| **AVERAGE NUTRITION FACTS:** | | | **1 bag (g)** | | |
|---|---|---|---|---|---|
| | **Per 100 g** | | **30** | **g** | ***NRV (%)*** |
| **Energy** | **1414** | kJ | 424 | kJ | |
| | **338** | kcal | 101 | kcal | |
| **Fat** | **4.89** | g | 1.47 | g | |
| *saturated* | *2.99* | *g* | *0.90* | g | |
| **Carbohydrate** | **2.33** | g | 0.70 | g | |
| *sugars* | *0.86* | *g* | *0.26* | *g* | |
| *polyols* | | *g* | *0.00* | *g* | |
| **Fiber** | **9.04** | g | 2.71 | g | |
| **Protein** | **66** | g | 20 | g | |
| **Salt** | **1.70** | g | 0.51 | g | |
| Vitamin C | 46.67 | mg | 14.00 | mg | *17.5* |
| Niacin | 9.33 | mg | 2.80 | mg | *17.5* |
| Vitamin E | 6.80 | mg | 2.04 | mg | *17.0* |
| Pantothenic acid | 3.53 | mg | 1.06 | mg | *17.7* |
| Riboflavin | 0.87 | mg | 0.26 | mg | *18.6* |
| Pyridoxine Hydrochloride | 0.83 | mg | 0.25 | mg | *17.9* |
| Thiamine Hydrochloride | 0.67 | mg | 0.20 | mg | *18.2* |
| Vitamin A | 0.50 | mg | 0.15 | mg | *18.8* |
| Folic acid | 0.13 | mg | 0.040 | mg | *20* |
| Biotin | 0.03 | mg | 0.009 | mg | *18* |
| Vitamin D3 | 0.0030 | mg | 0.0009 | mg | *18* |
| Vitamin B12 | 0.002 | mg | 0.0005 | mg | *20* |

### 6. High-protein powder for the preparation of a Berry flavored Dessert

INGREDIENTS: Milk Protein MPI (75.70%); Fruit powder and grains (8.69%): Raspberry, Blueberry; Soluble corn fiber Corn dextrin (4.71%); Tara gum (3.57%); Flavors; Silicon dioxide; Sodium chloride; L-Lysine; L-Tryptophan; Acidulants: Citric acid, Malic acid; Sucralose; L-ascorbic acid (Vit. C); DL-α-Tocopheryl acetate (Vit. E); Nicotinamide (Niacin); Retinyl acetate (Vit. A); Cholecalciferol (VIT .D); Calcium D-pantothenate (Pantothenic Acid); Riboflavin 5-phosphate (VIT. B2); Pyridoxine hydrochloride (Vit. B6); Thiamine hydrochloride (Vit. B 1); Cyanocobalamin (Vit. B 12); Pteroylmonoglutamic acid (Folic Acid); D-Biotin (Biotin).

| **AVERAGE NUTRITION FACTS:** | | | **1 bag (g)** | | |
|---|---|---|---|---|---|
| | **Per 100 g** | | **28** | **g** | ***NRV (%)*** |
| **Energy** | **1310** | kJ | 367 | kJ | |
| | **313** | kcal | 88 | kcal | |
| **Fat** | **1.25** | g | 0.35 | g | |
| *saturated* | *0.69* | *g* | *0.19* | *g* | |
| **Carbohydrate** | **3.44** | g | 0.96 | g | |
| *sugars* | 3.43 | *g* | *0.96* | *g* | |
| *polyols* | | *g* | | *g* | |
| **Fiber** | **7.25** | g | 2.03 | g | |
| **Protein** | **67** | g | 19 | g | |
| **Salt** | **1.16** | g | 0.32 | g | |
| Vitamin C | 50.00 | mg | 14.00 | mg | *17.5* |
| Niacin | 10.00 | mg | 2.80 | mg | *17.5* |
| Vitamin E | 7.29 | mg | 2.04 | mg | *17.0* |
| Pantothenic acid | 3.79 | mg | 1.06 | mg | *17.7* |
| Riboflavin | 0.93 | mg | 0.26 | mg | *18.6* |
| Pyridoxine Hydrochloride | 0.89 | mg | 0.25 | mg | *17.9* |
| Thiamine Hydrochloride | 0.71 | mg | 0.20 | mg | *18.2* |
| Vitamin A | 0.54 | mg | 0.15 | mg | *18.8* |
| Folic acid | 0.14 | mg | 0.040 | mg | *20* |
| Biotin | 0.03 | mg | 0.009 | mg | *18* |
| Vitamin D3 | 0.0032 | mg | 0.0009 | mg | *18* |
| Vitamin B12 | 0.002 | mg | 0.0005 | mg | *20* |

### 7. Highprotein powder useful for the preparation of a Cheese and Pepper flavored seasoning.

| | ***29 g bag*** | ***per 100 g*** |
|---|---|---|
| Milk protein MPI 90% | 16.15 | **55.70** |
| PEA PROTEIN 85% | 3.70 | **12.76** |
| Methionine | 0.44 | **1.51** |
| Threonine | 0.19 | **0.65** |
| Phenylalanine | 0.25 | **0.86** |
| Hydrated silica | 0.38 | **1.29** |
| Nutriose Fiber | 1.02 | **3.52** |
| Tara gum | 1.00 | **3.45** |
| Cheese powder | 3.75 | **12.93** |
| Aged cheese flavor | 1.13 | **3.88** |
| Pepper powder | 0.38 | **1.29** |
| Sodium chloride | 0.63 | **2.16** |
| | ***29.02*** | ***100.00*** |

### 8. High-protein powder useful for the preparation of a Pumpkin Flavored Velvety Soup

| | ***30 g bag*** | ***per 100 g*** |
|---|---|---|
| Milk protein MPI 90% | 10.84 | **36.13** |
| PEA PROTEIN 85% | 12.14 | **40.47** |
| Methionine | 0.40 | **1.32** |
| Phenylalanine | 0.12 | **0.40** |
| Threonine | 0.12 | **0.40** |
| Hydrated silica | 0.34 | **1.12** |
| Nutriose Fiber | 0.92 | **3.07** |
| Tara Gum | 1.00 | **3.33** |
| Pumpkin flavor | 1.20 | **4.00** |
| Milk cream flavor/Cream flavor | 0.20 | **0.67** |
| Pepper powder | 0.15 | **0.50** |
| Pumpkin powder | 0.80 | **2.67** |
| Beta carotene | 0.09 | **0.30** |
| Sucralose | 0.005 | **0.02** |
| Sodium chloride | 0.98 | **3.27** |
| Roast potato flavor | 0.40 | **1.33** |
| Natural onion flavor | 0.30 | **1.00** |
| | ***30.005*** | **100.00** |

### 9. Viscosity measurement

For the compositions of Examples 1, 5, 6 and 7, viscosity measurements of the product reconstituted in water were made both at room temperature (23-24°C) and under hot conditions (60-70°C).

1 bag of the reference composition was dispersed in 200 ml of water at a temperature of 23-24°C. The viscosity of the reconstituted product thus obtained was determined every 3 minutes starting from T0 and ending at T15, using the AMETEK BROOKFIELD Viscometer instrument Model DV1 featuring impeller 4, at 30 RPM.

The values for the composition of Example 1 (Custard flavor; 27g of powder) are as follows:

| TIME | **T0** | **T3** | **T6** | **T9** | **T12** | **T15** |
|---|---|---|---|---|---|---|
| MEASURE mPa*s | 3,330 | 3,513 | 3,847 | 3,847 | 4,087 | 4,280 |

The values for the composition of Example 5 (Cocoa flavor; 30g of powder) are as follows:

| TIME | **T0** | **T3** | **T6** | **T9** | **T12** | **T15** |
|---|---|---|---|---|---|---|
| MEASURE mPa*s | 1987 | 2637 | 2967 | 3210 | 5620 | 5780 |

The values for the composition of Example 6 (Berry flavor; 27g of powder) are as follows:

| TIME | **T0** | **T3** | **T6** | **T9** | **T12** | **T15** |
|---|---|---|---|---|---|---|
| MEASURE mPa*s | 4010 | 4170 | 4350 | 4490 | 4560 | 4710 |

In the case viscosity is measured under hot conditions, the compound is dissolved in 200 ml of water at a temperature of 23-24°C, heated for about 40 seconds in the microwave at a power of 800 w and brought to a temperature of 60 - 70°C. Under these temperature conditions, the viscosity is determined every 3 minutes, starting from T0 and ending at T15, and leaving the compound in a beaker at room temperature.

In this case, the AMETEK BROOKFIELD Viscometer instrument Model DV1 featuring impeller 4 at 10 RPM is used as well.

The values for the Velvety Soup powder (30 g) are as follows:

| TIME | **T0** | **T3** | **T6** | **T9** | **T12** | **T15** |
|---|---|---|---|---|---|---|
| MEASURE mPa*s | 13040 | 15140 | 16240 | 16520 | 17460 | 17840 |

## Claims

1. A high-protein composition in powder form, comprising
- proteins in a concentration range from 15% to 85% by weight, said proteins including or consisting of milk protein isolate (MPI),
- fibers in a concentration range from 2% to 14% by weight, said fibers including or consisting of resistant dextrins and/or polydextrose,
- thickeners in a concentration range from 0.5% to 8% by weight, the thickeners including or consisting of Tara gum,
in combination with any excipients and/or diluents.

2. The composition according to claim 1, wherein the proteins further comprise, in addition to milk protein isolate (MPI),
- plant-based proteins, and/or
- further animal-based proteins, preferably selected from milk protein concentrate (MPC), egg proteins, white egg proteins.

3. The composition according to claim 1 or 2, further comprising nutraceutical actives selected from the group consisting of: one or more B-group vitamins, vitamin C, E-group vitamins, A-group vitamins, one or more D-group vitamins, amino acids, peptides, plant-based extracts and combinations thereof.

4. The composition according to any one of claims from 1 to 3, **characterized by** a carbohydrate content of less than 10% by weight based on the total weight of the composition.

5. The composition according to any one of claims from 1 to 4, **characterized by** a fat content of less than 7% by weight based on the total weight of the composition.

6. The composition according to any one of claims from 1 to 5, for use as food supplement or as food for individuals treated with low-calorie diet.

7. A use of the composition according to any one of claims from 1 to 5 as food supplement or as food for healthy individuals on a balanced diet.

8. A food supplement or food comprising or consisting of the high-protein composition according to any one of claims from 1 to 5.

9. The food supplement or food according to claim 8, in the form of single-dose sachet or single-serving.

10. The food supplement or food according to claim 8 or 9, wherein a single-dose sachet or a single serving is **characterized by** a calorie content ranging from 45 to 120 Kcal.

11. The food supplement or food according to claim 9 or 10, wherein a single-dose sachet or a single serving contains
- MPI in amounts from 10 to 25 g
- resistant dextrins in amounts from 0.3 to 4.0 g
- tara gum in amounts from 0.3 to 4.0 g
- nutraceutical actives in amounts from 0.01 g to 0.4 g, wherein the nutraceutical actives are selected from the group consisting of: one or more B-group vitamins, vitamin C, one or more E-group vitamins, one or more A-group vitamins, one or more D-group vitamins, plant-based extracts and combinations thereof
- optionally, powdered fruits and/or dried fruit pieces in amounts from 0.4 to 3 g
- optionally, powdered vegetables and/or dried vegetable pieces in amounts from 1 to 15 g
- optionally, chocolate pieces or cocoa powder in amounts from 1 to 10 g.
